# EUROPEAN PATENT SPECIFICATION

(11) **EP 1 351 700 B1**
(45) Date of publication and mention of the grant of the patent: **08.12.2004**
(21) Application number: 02708297.3
(22) Date of filing: 07.01.2002
(51) Int. Cl.: A61K 31/7072, A61K 38/38, A61P 35/04

(54) **CONJUGATES OF NUCLEOSIDES WITH LACTOSAMINATED HUMAN ALBUMINE**
NUKLEOSIDE KONJUGIERT MIT MENSCHLICHEM LACTOSAMINIERTEN ALBUMIN
CONJUGUES HEPATOTROPES DE MEDICAMENTS ANTIBLASTIQUES EXECUTANT UNE CHIMIOTHERAPIE LOCO-REGIONALE DE MICROMETASTASES HEPATIQUES APRES LEUR ADMINISTRATION PAR VOIE VEINEUSE PERIPHERIQUE

(30) Priority: 10.01.2001 IT MI20010027
(43) Date of publication of application: 15.10.2003
(73) Proprietor: Universita' di Bologna, 40126 Bologna (IT)
(72) Inventor: FIUME, Luigi, I-40100 Bologna (IT); DI STEFANO, Giuseppina, I-40100 Bologna (IT); BUSI, Corrado, I-40100 Bologna (IT)
(74) Representative: Pistolesi, Roberto
(86) International application number: PCT/EP2002/000435
(87) International publication number: WO 2002/055097

(56) References cited:
- US-A- 5 594 110
- FIUME L ET AL: "Liver targeting of nucleoside analogues coupled to galactosyl terminating macromolecules: a new approach to the treatment of a chronic viral hepatitis." ITALIAN JOURNAL OF GASTROENTEROLOGY AND HEPATOLOGY. ITALY JUN 1997, vol. 29, no. 3, June 1997 (1997-06), pages 275-280, XP008004174 ISSN: 1125-8055
- FIUME L ET AL: "Liver targeting of antiviral nucleoside analogues through the asialoglycoprotein receptor." JOURNAL OF VIRAL HEPATITIS. ENGLAND 1997, vol. 4, no. 6, 1997, pages 363-370, XP008004173 ISSN: 1352-0504
- FIUME L ET AL: "The pathogenesis of vacuoles produced in rat and mouse liver cells by a conjugate of adenine arabinoside monophosphate with lactosaminated albumin." JOURNAL OF HEPATOLOGY, vol. 15, no. 3, 1992, pages 314-322, XP008004165 ISSN: 0168-8278
- FIUME L ET AL: "Coupling of antiviral nucleoside analogs to lactosaminated human albumin by using the imidazolides of their phosphoric esters." ANALYTICAL BIOCHEMISTRY. UNITED STATES 1 AUG 1993, vol. 212, no. 2, 1 August 1993 (1993-08-01), pages 407-411, XP000371938 ISSN: 0003-2697
- PONZETTO A ET AL: "ARA AMP AND ACYCLOVIR MONOPHOSPHATE COUPLED TO LACTOSAMINATED ALBUMIN REDUCE WOODCHUCK HEPATITIS VIRUS VIREMIA AT DOSES LOWER THAN DO THE UNCONJUGATED DRUGS" HEPATOLOGY, vol. 14, no. 1, 1991, pages 16-24, XP000800471 ISSN: 0270-9139
- DI STEFANO G ET AL: "Enhanced liver blood concentrations of adenine arabinoside accomplished by lactosaminated poly-L-lysine coupling: implications for regional chemotherapy of hepatic micrometastases." BIOCHEMICAL PHARMACOLOGY. ENGLAND 1 FEB 2000, vol. 59, no. 3, 1 February 2000 (2000-02-01), pages 301-304, XP001074753 ISSN: 0006-2952
- FIUME L.; BUSI C.; DI STEFANO G.; MATTIOLI A.: 'Targeting of antiviral drugs to the liver using glycoprotein carriers' ADVANCED DRUG DELIVERY REVIEWS vol. 14, 1994, AMSTERDAM, pages 51 - 65

## Description

The present invention refers to pharmaceutical compositions containing as active compound conjugates of antiblastic nucleosides (and their analogs), preferably FUdR, with lactosaminated albumin (L-SA) and, more particularly, with lactosaminated human albumin (L-HSA). These conjugates are particularly useful to increase the efficacy of the antiblastic nucleosides (and their analogs) on liver micrometastases (avascular metastases nourished by the blood of liver sinusoids). The conjugates selectively enter into hepatocytes and release the drugs into liver blood in pharmacologically active amounts.

### Technical field of the invention

Colorectal cancer is one of the most common malignant disease with about half million deaths and more than 700,000 new cases diagnosed worldwide each year (Pisani P., et al. "Estimates of the world wide mortality..." Int J Cancer 1993, 55, 891-903). Hepatic metastases develop in 60 % of patients with colorectal cancer and autopsy studies have shown that metastatic disease remains confined to the liver in a third of patients who die of this tumor (Kemeny N., et al. "Hepatic arterial infusion..." New Engl J Med 1999, 341, 2039-2048). Fluoropyrimidines (5-fluoro-2'-deoxyuridine (FUdR) and its precursor 5-fluorouracil (FU)) are the drugs of choice for the post-operative chemotherapy of metastases from colon carcinoma (Robustelli Della Cuna G., Gennari L. "Neoplasie dell'apparato digerente" in Bonadonna G., Robustelli Della Cuna G. "Medicina Oncologica", Masson, Milano, Parigi, 1999). Fluoropyrimidines are characterised by high hepatic extraction: in patients receiving these drugs by peripheral venous administration, the levels of FUdR (Ensminger W.D., et al. "A clinical-pharmacological evaluation..." Cancer Res 1978, 38, 3784-3792) and of FU (Wagner J.G., et al. "Steady-state non linear pharmacokinetics..." Cancer Res 1986, 46, 1499-1506) are lower in hepatic veins than in systemic circulation. This extraction causes cells of liver micrometastases (early metastases nourished by the blood of liver sinusoids) to be exposed to drug concentrations lower than those achievable in systemic circulation. This is a serious disadvantage, since micrometastases should be one of the major targets of post-operative chemotherapy. To increase the efficacy of fluoropyrimidines on liver micrometastases these drugs were locally administered through portal vein (Taylor I., et al. "Randomised controlled trial..." Br J Surg 1985, 72, 359-363; Fielding L.P., et al. "Randomised controlled trial..." Lancet 1992, 340, 502-506; Liver Infusion Meta-analysis Group "Portal vein chemotherapy..." J Natl Cancer Inst 1997, 89, 497-505; Rougier P., et al. "Adjuvant portal vein infusion..." Lancet 1998, 351, 1677-1681). However, portal vein infusion requires laparatomy and the inserted catheter often causes complications.

In recent years, it was observed that nucleoside analogs (NAs) (the chemical family of FUdR) when coupled to peptides exposing galactosyl residues (e.g. lactosaminated poly-L-lysine (L-poly(LYS)) selectively enter into hepatocytes after binding to the asialoglycoprotein receptor expressed only on hepatocyte surface (Fiume L., et al. "Liver targeting..." J Viral Hep 1997, 4, 363-370). It has been reported that adenine arabinoside monophosphate (ara-AMP) can be coupled to lactosaminated human albumin and used for the treatment of chronic viral hepatitis (Fiume L. and Verme G. "Liver targeting of nucleoside analogues..." Ital. J. Gastroenterol. Hepatol. 1997, 29, 275-80; Fiume L., et al. "The pathogenesis of vacuoles ..." Journal of Hepatology, 1992, 15, 314-322; Fiume L., et al. "Coupling of antiviral ... " Analytical Biochemistry, 1993, 212, 407 - 411). It was found that, after the intracellular release from the carrier, NAs partly exit from hepatocytes into bloodstream (Fiume L., et al. "Coupling to lactosaminated poly-L-lysine..." J Hepatol 1998, 29, 1032-1033; Di Stefano G., et al. "Inhibition of [³H]thymidine incorporation..." Biochem Pharmacol 1999, 57, 793-799). Although this exit reduces the efficacy of hepatocyte targeting, it may have a useful consequence, since it results in higher NA concentrations in hepatic blood than in systemic circulation (Di Stefano G., et al. "Enhanced liver blood concentrations..." Biochem Pharmacol 2000, 59, 301-304). Therefore, administration of antiblastic nucleosides (and their analogs) coupled to galactosylated peptides, such as L-poly(LYS), could reproduce the loco-regional chemotherapy performed by the intra-portal infusion of fluoropyrimidines with the advantage of avoiding the drawbacks of this invasive procedure and permitting repeated cycles of treatment. The feasibility of this approach received support by the finding that in mice a conjugate of FUdR with L-poly(LYS) released the drug in liver blood in amounts high enough to inhibit the growth of hepatic metastases (Di Stefano G., et al., "Coupling of fluorodeoxyuridine to lactosaminated..." Biochem Pharmacol 2001, 61, 457-463).

Conjugates prepared with L-poly(LYS) have an important drawback which hinder their clinical use. With the available procedures of synthesis, poly(LYS) is composed of molecules with different molecular weights, whose proportion and range vary according to the different preparations. As a consequence, the amounts of L-poly(LYS)-FUdR conjugate eliminated through the kidney change with the batch of poly(LYS) used (Di Stefano G., et al., unpublished), so that different pharmacokinetics and active doses are to be expected.

### Description of the invention

The drawback of L-poly(LYS) (molecular weight heterogeneity of the peptide backbone) is not displayed by lactosaminated albumin (L-SA), a hepatotropic carrier in which the galactosyl residues are introduced in the albumin molecule (Fiume L., et al. "Hepatocyte targeting of adenine-9-β-D-arabinofuranoside..." FEBS Lett 1981, 129, 261-264). On the other hand, the conjugates of L-SA have a molar ratio drug / carrier several times lower than that of the L-poly(LYS) conjugates (Di Stefano G., et al. "Selective delivery to the liver of antiviral..." Biochem Pharmacol 1995, 49, 1769-1775). As a consequence, when the hepatic asialoglycoprotein receptor is saturated (Fiume L., et al. "Lactosaminated human serum albumin..." FEBS Lett 1982, 146, 42-46) and the number of conjugate molecules entering hepatocytes can not be increased, the drug amounts introduced in these cells (and those which exit in bloodstream) are much lower after the administration of L-SA conjugates. For this reason, a pharmacological action in liver blood accomplished by L-SA conjugates could not be anticipated.

In the experiments here described, we found that amounts of drug pharmacologically active on liver metastases are released in liver blood even after the administration of a L-SA-FUdR conjugate, in spite of its molar ratio 7-8 fold lower than that of L-poly(LYS)-FUdR. Therefore, L-SA-FUdR possesses the same potentiality as L-poly(LYS)-FUdR for increasing the therapeutic efficacy of FUdR against liver micrometastases, having the advantage of assuring a reproducible renal elimination and pharmacokinetics among the various conjugate preparations.

Therefore the object of the present invention is represented by therapeutical compositions containing as active drug conjugates of antiblastic nucleosides (or their analogs) with lactosaminated albumin (L-SA) and, in particular, human lactosaminated albumin (L-HSA),

Moreover, a second object of the invention is represented by the use of conjugates of antiblastic nucleosides (or their analogs) with lactosaminated albumin (L-SA) and, in particular, human lactosaminated albumin (L-HSA) for the preparation of therapeutical compositions with an increased efficacy on liver micrometastases.

Preferably, the therapeutical compositions according to the present invention are aqueous solutions administrable by parenteral route, preferably by intravenous route, by bolus or by infusion; in addition to the conjugates above described, they may obviously contain common excipients and adjuvants known in the art

In the present invention, the most preferred antiblastic nucleoside is 5-fluoro 2'-deoxyuridine (FUdR).

The compositions according to the present invention may be used for increasing the efficacy of chemotherapeutical treatments on hepatic micrometastases in mammalian and, in particular, in human beings.

These and other aspects of the invention will be evident in view of the following experimental part.

### MATERIALS AND METHODS

### Preparation and characterisation of L-HSA-FUdR

α-Lactose was coupled to ε-NH₂ lysine residues of HSA (crystallized, essentially globulin free) by reductive amination (Wilson G. "Effect of reductive lactosamination..." J Biol Chem 1978, 253, 2070-2072). The lactose/HSA molar ratio, measured as described (Fiume L., et al. "Selective penetration and pharmacological..." Gut 1984, 25, 1392-1398), was 24. Conjugation of FUdR was obtained via the imidazolide of its 5'-phosphoric ester (Fiume L., et al. "Coupling of antiviral nucleoside analogs..." Anal Biochem 1993, 212, 407-411). Using this procedure, NAs are linked to lysine residues of L-HSA by a phosphate bridge. FUdR was phosphorylated in the primary OH group according to Yoshikawa M. et al. ("A novel method for phosphorylation..." Tetrahedron Lett 1967, 50, 5065-5068). After the extraction of trimethyl phosphate with chloroform and neutralization with NaOH, FUdR monophosphate (FUdRMP) was purified by chromatography on a DEAE Sephadex column eluted with an ammonium bicarbonate gradient (250 - 500 mM). Fractions containing FUdRMP were concentrated under reduced pressure and lyophilised. FUdRMP was converted into its imidazolide (FUdRMPlm) according to Lohrman R. and Orgel L.E. ("Preferential formation of (2'-5')-linked..." Tetrahedron 1978, 34, 853-855), using dimethylsulphoxide instead of dimethylformamide as solvent. In a typical conjugate preparation, 100 mg L-HSA and 200 mg FUdRMPIm were dissolved in 2 ml 0.1M sodium carbonate buffer, pH 9.5. After 48 h at 37°, the conjugate was dialysed against water and lyophilised. The FUdR/L-HSA molar ratio was spectrophotometrically determined as described (Fiume L., et al. " Drug targeting in antiviral..." Biochem Pharmacol 1986, 35, 967-972). Electrophoretic analysis of the conjugate was performed following the method of Weber K. and Osborn M. ("The reliability of molecular weight..." J Biol Chem 1969, 244, 4406-4412), using gels containing 5% acrylamide, 0.07% methylene bisacrylamide and 0.3% sodium dodecyl sulphate. After staining with Coomassie R250 blue gels were photographed and the densitometric analysis of bands was performed using the GelPro Analyser 3.0 software (Media Cybernetics; Silver Spring, MD).

Two radioactive conjugates were synthesised: one labelled in the carrier (L-[¹⁴C]HSA-FUdR) and one in the drug moiety (L-HSA-[³H]FUdR). The former (two preparations) was obtained by labelling L-HSA with [¹⁴C]-formaldehyde (56 mCi/mmol), according to Jentoft N. and Dearborn D.G. ("Protein labelling by reductive..." Methods Enzymol 1983, 91, 570-579). The conjugate radioactive in the drug moiety (three preparations) was obtained using [³H]FUdR (Moravek). Specific activity of conjugated [³H]FUdR ranged from 4.3 to 4.6 x 10⁴ dpm/µg.

### Animals

Female Balb/C mice 7-8 weeks old (weighing 16-19 g) and male Wistar rats (weighing 170-190 g) were used. They were obtained from Harlan Italy and were maintained in an animal facility at the Department of Experimental Pathology, Bologna, receiving humane care, in accordance with European Legislation. The protocols of the experiments were approved by the Ethical Committee of the University of Bologna. Animals were fed a standard pellet diet *ad lib.*

### Blood sampling from inferior vena cava and hepatic veins of rats

Blood sampling from inferior vena cava and hepatic veins of rats was performed as described by Di Stefano G., et al. ("Enhanced liver blood concentrations..." Biochem Pharmacol 2000, 59, 301-304).

### Determination of [³H]FUdR in plasma

Plasma levels of free [³H]FUdR were measured by using the isotope dilution procedure and HPLC. In order to identify the elution time of [³H]FUdR in HPLC chromatogram and to evaluate the recovery, 15 µg of unlabeled FUdR was added to each plasma sample (400 µl), kept cold in ice. Plasma proteins were removed by the addition of 30 µl trichloroacetic acid 80% and centrifugation at 2-4°C. After diethylether extraction to eliminate trichloroacetic acid, 200 µl supernatant was chromatographed on a Spherisorb ODS2 equilibrated on 20 mM sodium tetraborate pH 7.5 and eluted with a linear gradient of methanol (0 - 30 %). Radioactivity eluting at the position of FUdR marker was counted and the plasma concentration of [³H]FUdR was calculated taking into account the recovery of the marker (measured by UV absorbance) and the specific activity (SA) of injected [³H]FUdR. The recovery of the FUdR marker was 85-95%. The SA of free or conjugated [³H]FUdR ranged from 4.3 to 4.6 x 10⁴ dpm/µg. When radioactivity of FUdR peak was lower than 200 dpm, the plasma concentration of [³H]FUdR was considered below a measurable level (BML). With a 90% recovery of FUdR marker, the lowest measurable plasma concentration was about 25 ng [³H]FUdR/ml.

### EXPERIMENTAL OBSERVATIONS

L-HSA-FUdR easily dissolved in NaCl 0.9 % at 300 mg/ml. The molar ratio FUdR / L-HSA ranged from 14 to 16 in ten different preparations. It was 7-8 fold lower than that of L-poly(LYS)-FUdR (Di Stefano G., et al. "Coupling of 5-fluoro-2'-deoxyuridine..." Biochem Pharmacol 2001, 61, 457-463). SDS polyacrilamide gel electrophoresis showed that FUdR coupling did not cause any covalent cross-linking of L-HSA.

Table 1 shows that L-[¹⁴C]HSA-FUdR, injected intravenously into mice and rats, was selectively taken up by the liver.

**TABLE 1**

| Radioactivity in plasma and organs of mice and rats after intravenous injection of L-[¹⁴C]HSA-FUdR | | | | | | | |
|---|---|---|---|---|---|---|---|
| **Animal** | **Dose (µg/g)** | **Time (h)** | | **Radioactivity (dpm /g /SA)**^{**a**}**)** | | | |
| | | | **Plasma** | **Liver** | **Kidney** | **Spleen** | **Intestine** |
| **Mouse** | 13^{b}) | 0.25 | 71.6±2.2 | 106.1±5.6 | 3.8±0.2 | 7.4±0.4 | 3.4±0.5 |
| | 13 | 0.5 | 14.6±0.5 | 177.1 ±10.2 | 9.1 ± 0.5 | 5.9±0.6 | 4.6±0.6 |
| | 13 | 1 | 3.2±0.1 | 143.618.1 | 11.3 ± 1.3 | 7.6±1.2 | 6.9 ± 1.3 |
| | 13 | 2 | 1.2±0.1 | 137.1±13.0 | 10.2±0.9 | 5.3±0.7 | 4.7±0.4 |
| | 26^{b}) | 0.5 | 331.7±2.1 | 577.8 ± 15.6 | 50.6±14.3 | 45.7±14.1 | 25.3±1.2 |
| | 26 | 1 | 19.5±3.0 | 849.8±52.7 | 58.5±3.9 | 53.8±1.9 | 28.6±4.2 |
| | 26 | 2 | 10.3 ± 1.8 | 645.9 ± 82.7 | 52.9 ± 4.9 | 36.5 ± 4.6 | 20.7 ± 5.5 |
| **Rat** | 13 | 0.5 | 42.3±16.8 | 140.4±7.6 | 14.3±2.1 | 3.7±0.2 | 3.9±0.2 |
| | 13 | 1 | 1.5±0.1 | 133.7±9.8 | 18.0±1.4 | 6.7±0.2 | 4.7±0.6 |
| | 13 | 2 | 1.7 ± 0.1 | 1 09.4 ±5.4 | 20.9 ±3.2 | 5.5 ±0.8 | 4.8 ±0.3 |

| | | | | | | | |
|---|---|---|---|---|---|---|---|
| Animals were intravenously injected with a conjugate labelled in the carrier moiety (L-[¹⁴C]HSA-FUdR). Organ distribution of radioactivity was measured as described by Fiume L., et al. ("Lactosaminated human serum albumin..." FEBS Lett 1982, 146, 42-46). The radioactive contribution given by the plasma trapped in the organs of mice (Fiume L., et al. "Lactosaminated human serum albumin..." FEBS Lett 1982, 146, 42-46) and rats (Fiume L., et al. "Distribution of a conjugate..." Cancer Drug Deliv 1987, 4, 11-16) was subtracted. Data are mean values ± SE from 2-3 animals. *a* Reported values are total (acid soluble + insoluble) radioactivity for organs. Acid insoluble radioactivity is given for plasma. SA = Specific Activity (1.4-1.6 x 10³ dpm/µg). | | | | | | | |
| *b* 13 and 26 µg of L-[¹⁴C]HSA-FUDR contained 0.6 and 1.2 µg FUdR, respectively. | | | | | | | |

Table 2 shows the effect of free and conjugated FUdR on the growth of hepatic metastases induced in mice by intrasplenic injection of murine colon carcinoma C-26 cells. Compounds were injected intravenously every other day, starting 24 h after inoculation of tumour cells. Four administrations were performed, and the animals were sacrificed one day after the last injection. Free FUdR inhibited the tumour growth at the daily dose of 10 µglg, whereas conjugated FUdR was active at a dose 16-17 times lower (0.6 µg/g).

**TABLE 2**

| Effect of free and conjugated FUdR on liver metastases of C-26 cells in mouse | | | | |
|---|---|---|---|---|
| **Exp.** | **Compound** | **Daily Dose (µg/g)** | **Increase of liver weight (9)**^{**a)**} | **Total Liver DNA (mg)** |
| 1 | Saline | | 1.22 ± 0.25 | 11.83±1.75 |
| | Free FUdR | 5 | 1.00±0.17 NS | 9.47±1.06 NS |
| | | 10 | 0.64±0.15 p=0.070 | 7.32±0.91 p = 0.041 |
| 2 | Saline | | 1.52±0.14 | 13.27±0.73 |
| | Coupled FUdR | 0.3 | 1.24 ±0.26 NS | 10.55 ±1.09 NS |
| | | 0.6 | 0.87±0.09 p =0.002 | 6.47 ±0.37 p = 0.000 |
| 3 | Saline | | 1.37±0.09 | 11.99±0.78 |
| | Coupled FUdR | 1.2 | 0.95 ± 0.09 p = 0.008 | 8.99 ± 1.00 p = 0.036 |

| | | | | |
|---|---|---|---|---|
| Liver metastases were obtained as described (Di Stefano G., et al. "Coupling of 5-fluoro-2'-deoxyuridine..." Biochem Pharmacol 2001, 61, 457-463). Seven animals were used for each treatment group. The mass of the metastases was estimated by measuring the increase in liver weight and by determining the total hepatic DNA which was extracted by the method of Schneider W.C. and Greco A.E. ('Incorporation of pyrimidine deoxyribonucleosides..." Biochim Biophys Acta 1971, 228, 610-626) and measured according to Burton K. ("A study of the conditions..." Biochem J 1956, 62, 315-323). Data are mean values ± SE. Data were statistically evaluated by Student's t-test. In preliminary experiments, we found that total DNA content of normal liver was 2.69 ± 0.13 mg; it increased to 4.24 ± 0.38 and to 13.03 ± 0.82 mg, 5 and 8 days, respectively, after tumour transplant. These values (mean ± SE) were obtained using 5 animals for each group. *a* The weight of the liver was measured and that of non tumour-bearing Balb/C mice (0.90 ± 0.02 g) was subtracted. | | | | |

To verify whether the selective delivery of FUdR to hepatic cells accomplished by the conjugate causes liver damage, we compared the effect of free and conjugated FUdR on the serum alanine aminotransferase (ALT) (EC 2.6.1.2) level in mice (Table 3). An increase in the level of this enzyme is an index of hepatocyte damage. The drugs were administered according to the schedule used for the experiments on the growth of hepatic metastases. The conjugated drug at a dose 5 times higher than that active on the metastases had no effect; at a dose 10 times higher it produced a statistically non-significant increase of ALT level. The free drug at a dose 5 times higher than that active on hepatic metastases caused an increase in ALT levels at the limit of statistical significance (p = 0.041).

**TABLE 3**

| Effect of free and L-HSA coupled FUdR on alanine aminotransferase (ALT) serum levels in mouse | | | |
|---|---|---|---|
| **Exp** | **Compound** | **Dose (µg/g)** | **Serum ALT levels (U/I)** |
| 1 | Saline | | 110.4±22.1 |
| | Coupled FUdR | 3 | 84.4±10.6 NS |
| 2 | Saline | | 64.5 ± 15.0 |
| | Coupled FUdR | 6 | 112.7±18.7 NS |
| | Free FUdR | 50 | 114.8±16.0 p = 0.045 |
| Mice received four intravenous injections of the compounds on alternate days. Six animals were used in each group. Serum ALT activity was determined the day after the last administration using a kit available from Roche Diagnostics. Data are mean values ± SE. Results were statistically evaluated by Student's t-test. | | | |

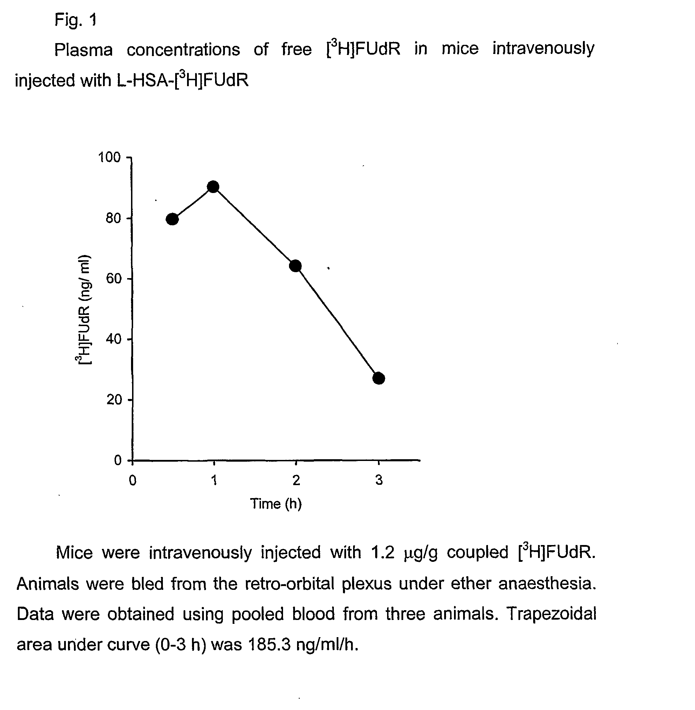

Figure 1 shows [³H]FUdR plasma levels in mice intravenously injected with 1.2 µg/g of coupled [³H]FUdR.

Table 4 shows concentrations of free [³H]FUdR in inferior vena cava (IVC) and in hepatic veins (HV) of rats injected intravenously with the free or conjugated drug. These concentrations are a measure of drug levels in systemic circulation and in liver blood, respectively. In rats injected with the conjugate the ratios between [³H]FUdR levels in HV and those in IVC were 6-7 times higher than in animals administered with the free drug. This result demonstrates that in animals injected with the conjugate the release of FUdR in bloodstream occurs in liver.

The finding that in rats injected with the free drug, [³H]FUdR levels in HV are several times lower than those in IVC is in agreement with data in patients and is explained by the capacity of hepatocytes of extracting fluoropyrimidines from blood (Ensminger W.D., et al. "A clinical-pharmacological evaluation..." Cancer Res 1978, 38, 3784-3792; Wagner J.G., et al. "Steady-state non-linear pharmacokinetics..." Cancer Res 1986, 46, 1499-1506) . In systemic chemotherapy with these drugs, hepatic extraction causes cells of liver micrometastases to be exposed to drug concentrations lower than those achievable in systemic circulation. This is a serious disadvantage, since micrometastases should be one of the major targets of post-operative adjuvant chemotherapy. Our results suggest that this drawback might be overcome by FUdR coupling with L-HSA.

In patients with colorectal cancer who received an intravenous continuous infusion of a therapeutic dose of FUdR (Chang A.E., et al. "A prospective randomised trial..." Ann Surg 1987, 206, 685-693; Kemeny N., et al. "Intrahepatic or systemic infusion of fluorodeoxyuridine..." Ann Int Med 1987, 107, 459-465), the estimated blood concentration of the drug was very low (about 0.2 ng/ml) (Park J.G., et al. "Enhancement of fluorinated pyrimidine-induced..." J Natl Cancer Inst 1988, 80, 1560-1564). Such a concentration could be easily achieved in patients administered with L-HSA-FUdR, considering the much higher drug levels we measured in the systemic circulation of mice and rats injected with this conjugate (Figure 1 and Table 4).

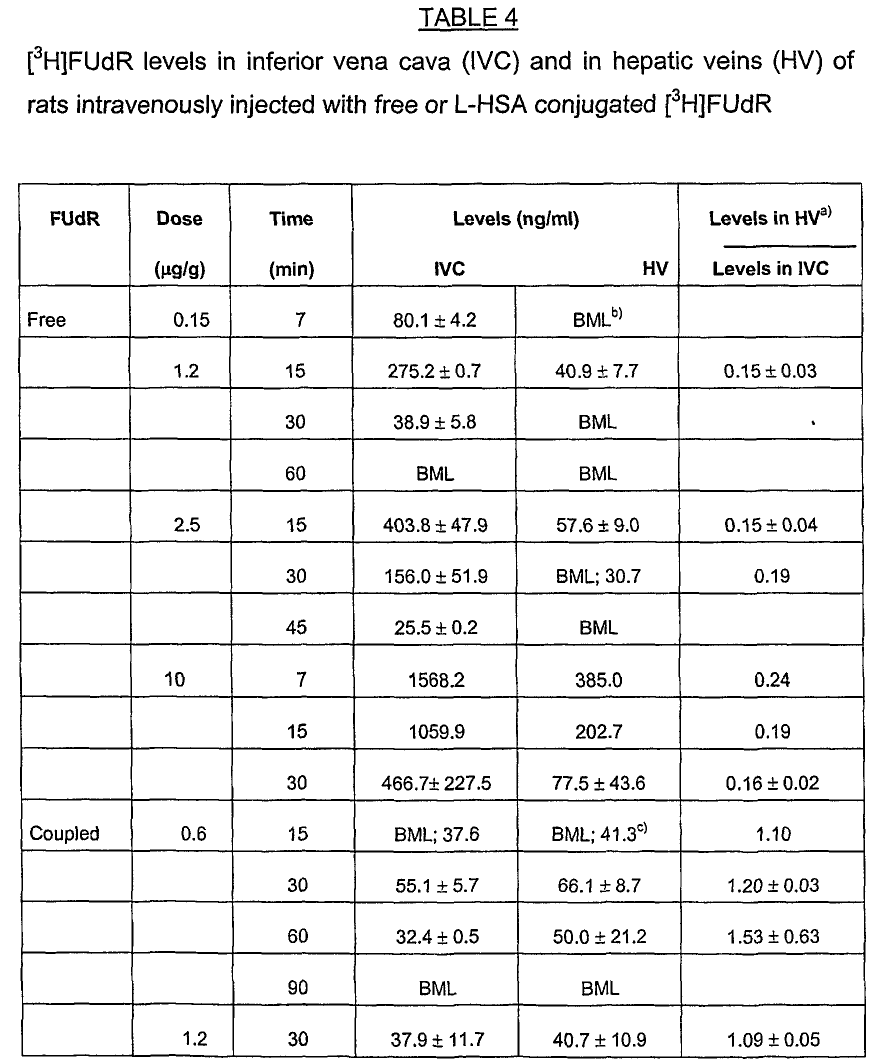

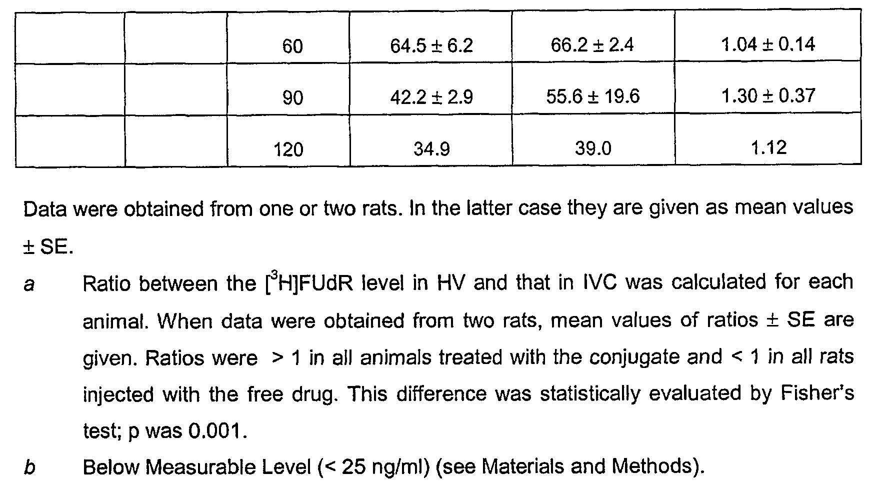

### CONCLUSIONS

The above reported experiments demonstrate that L-HSA-FUdR conjugate, in spite of its drug / carrier molar ratio 7-8 times lower than that of L-poly(LYS)-FUdR, releases FUdR in bloodstream in amounts high enough to inhibit the growth of hepatic metastases in mice. Moreover, it produces FUdR levels in the systemic circulation of mice and rats which are even higher than those pharmacologically active in patients treated with this drug (Park J.G., et al. "Enhancement of fluorinated pyrimidine-induced..." J Natl Cancer Inst 1988, 80, 1560-1564). The experiments reported in Table 4 show that, at the same concentrations of FUdR in systemic circulation, the levels of drug in liver blood were several times higher when FUdR was administered to rats in the conjugated form. Therefore, L-HSA-FUdR conjugate has the potentiality to increase the efficacy of fluoropyrimidines in the adjuvant chemotherapy of tumors giving hepatic metastases. Administration of the conjugate should lead to higher FUdR concentrations locally in liver blood, while at the same time producing systemic drug levels which can be pharmacologically active. Therefore the grant of a patent for the use of L-HSA-FUdR conjugates in the post-operative chemotherapy of the colon rectal tumor and, more generally, of hepatic metastatic tumors, sensitive to fluoropyrimidine is required.

## Claims

1. Therapeutical compositions comprising conjugates of 5-fluoro-2'-deoxyuridine with lactosaminated albumin as active compound in admixture with a pharmaceutically acceptable carrier.

2. Composition according to claim 1 **characterized in that** the lactosaminated albumin is human lactosaminated albumin.

3. Composition according to claims 1-2 **characterized in that** said composition is an aqueous solution administrable by parenteral route.

4. Composition according to claim 3 **characterized in that** said composition is administrable by intravenous route, by bolus or by infusion.

5. Composition according to claims 1-5 **characterized in that** said composition includes common excipients and adjuvants.

6. Use of conjugates of 5-fluoro-2'-deoxyuridine with lactosaminated albumin for manufacturing therapeutical compositions for the treatment of hepatic micrometastases.

7. Use according to claim 6 **characterized in that** lactosaminated albumin is human lactosaminated albumin.

8. Use according to claims 6-7 **characterized in that** said therapeutical compositions are aqueous solutions administrable by parenteral route.

## Patentansprüche

1. Therapeutische Zusammensetzungen umfassend Konjugate aus 5-Fluor-2'-deoxyuridin mit laktosaminiertem Albumin als aktive Verbindung in Mischung mit einem pharmazeutisch annehmbaren Träger.

2. Zusammensetzung gemäß Anspruch 1, **dadurch gekennzeichnet, daß** das laktosaminierte Albumin menschliches laktosaminiertes Albumin ist.

3. Zusammensetzung gemäß Ansprüchen 1 bis 2, **dadurch gekennzeichnet, daß** die Zusammensetzung eine wäßrige Lösung ist, die auf parenteralem Wege verabreichbar ist.

4. Zusammensetzung gemäß Anspruch 3, **dadurch gekennzeichnet, daß** die Zusammensetzung auf intravenösem Wege, als Bolus oder durch Infusion verabreichbar ist.

5. Zusammensetzung gemäß Anspruch 1 bis 4, **dadurch gekennzeichnet, daß** die Zusammensetzung gewöhnliche Trägerstoffe und Adjuvantien einschließt.

6. Verwendung von Konjugaten aus 5-Fluor-2'-deoxyuridin mit laktosaminiertem Albumin zur Herstellung therapeutischer Zusammensetzungen zur Behandlung hepatischer Mikrometastasen.

7. Verwendung gemäß Anspruch 6, **dadurch gekennzeichnet, daß** das laktosaminierte Albumin menschliches laktosaminiertes Albumin ist.

8. Verwendung gemäß Ansprüchen 6 bis 7, **dadurch gekennzeichnet, daß** die therapeutischen Zusammensetzungen wäßrige Lösungen sind, die auf parenteralem Wege verabreichbar sind.

## Revendications

1. Compositions thérapeutiques comprenant des conjugués de 5-fluoro-2'déoxyuridine comportant de l'albumine lactosaminée comme composé actif mélangée à un véhicule pharmaceutiquement acceptable.

2. Composition selon la revendication 1, **caractérisée en ce que** l'albumine lactosaminée est de l'albumine lactosaminée humaine.

3. Composition selon les revendications 1 à 2, **caractérisée en ce que** ladite composition est une solution aqueuse destinée à être administrée par voie parentérale.

4. Composition selon la revendication 3, **caractérisée en ce que** ladite composition est destinée à être administrée par voie intraveineuse, par bolus ou par perfusion.

5. Composition selon les revendications 1 à 5, **caractérisée en ce que** ladite composition comprend des excipients et des adjuvants communs.

6. Utilisation de conjugués de 5-fluoro-2'déoxyuridine comportant de l'albumine lactosaminée pour fabriquer des compositions thérapeutiques destinées au traitement de micrométastases hépatiques.

7. Utilisation selon la revendication 6, **caractérisée en ce que** l'albumine lactosaminée est de l'albumine lactosaminée humaine.

8. Utilisation selon les revendications 6 à 7, **caractérisée en ce que** lesdites compositions thérapeutiques sont des solutions aqueuses destinées à être administrées par voie parentérale.
